# EUROPEAN PATENT SPECIFICATION

(11) **EP 1 408 940 B1**
(45) Date of publication and mention of the grant of the patent: **17.09.2008**
(21) Application number: 02745700.1
(22) Date of filing: 18.07.2002
(51) Int. Cl.: A61K 31/135, A61P 25/16, A61P 25/28, A61P 25/14

(54) **PHARMACEUTICAL COMPOSITION COMPRISING DERAMCICLANE FOR THE TREATMENT OF THE DECLINE AND/OR DAMAGE OF COGNITIVE FUNCTIONS**
DERAMCICLAN-ENTHALTENDE PHARMAZEUTISCHE ZUSAMMENSETZUNG FÜR DIE BEHANDLUNG DER VERRINGERUNG KOGNITIVER FUNKTIONEN UND/ODER SCHÄDEN DAVON
COMPOSITION PHARMACEUTIQUE POUR LE TRAITEMENT DU DECLIN ET/OU DES ATTEINTES DES FONCTIONS COGNITIVES

(30) Priority: 18.07.2001 HU 0103017
(43) Date of publication of application: 21.04.2004
(73) Proprietor: EGIS Gyógyszergyár Nyrt, 1106 Budapest (HU)
(72) Inventor: GACSALYI, István, H-1021 Budapest (HU); LEVAY, György, Gàbor ron u. 10., H-2092 (HU); NAGYNE GYÖNÖS, Ildiko, H-1192 Budapest (HU); HARSING, Lászlo, Gábor, H-1071 Budapest (HU)
(74) Representative: Beszédes, Stephan G.
(86) International application number: PCT/HU2002/000069
(87) International publication number: WO 2003/007926

(56) References cited:
- WO-A-02/43726
- US-A- 4 342 762
- US-A- 6 093 747
- KANERVA H ET AL: "PHARMACOKINETICS AND SAFETY OF DERAMCICLANE DURING MULTIPLE ORAL DOSING" INTERNATIONAL JOURNAL OF CLINICAL PHARMACOLOGY AND THERAPEUTICS, DUSTRI-VERLAG, DEISENHOFEN-MUENCHEN, DE, vol. 37, no. 12, December 1999 (1999-12), pages 589-597, XP000937794 ISSN: 0946-1965
- KANERVA H ET AL: "THE SINGLE DOSE PHARMACOKINETICS AND SAFETY OF DERAMCICLANE IN HEALTHY MALE VOLUNTEERS" BIOPHARMACEUTICS AND DRUG DISPOSITION, WILEY, CHICHESTER, US, vol. 20, no. 7, October 1999 (1999-10), pages 327-334, XP000937808 ISSN: 0142-2782

## Description

### Field of the invention

The present invention relates to the use of a bicycloheptane derivative for the preparation of medicaments for the treatment or prophylaxis of diseases characterized by either the decline and/or damage of cognitive functions, particularly to a pharmaceutical composition for the treatment of Korsakoff syndrome, Huntington's disease, Alzheimer disease or dementia.

### Technical background

It is known that 1,7,7-trimethylbicyclo[2.2.1]heptane derivatives bearing a phenyl, phenyl-alkyl, or thienyl group and a dialkylaminoalkyl side chain in position 2 display anticonvulsive, motor-depressant, analgetic effect, and potentiate hexobarbital-induced narcosis (GB Patent No. 2 065 122). One of the prominent representatives of this compound group is (1R,2S,4R)-(-)-2-(2-dimethylaminoethoxy)-2--phenyl-1,7,7-trimethylbicyclo[2.2.1]heptane, known as deramciclane, in form of free base and its pharmaceutically acceptable acid addition salts, particularly the fumarate salt. Such compounds are disclosed in Hungarian Patent No. 212 547.

Deramciclane displayed significant effect on the animal models of anxiety and depression. In the Vogel lick-conflict model, deramciclane was effective after 1 and 10 mg/kg per os treatment in rats. In the social interaction model [File S.E.J. Neurosci. Methods, 2, 219-238 (1980)], deramciclane increased the number of social interactions following as low as 0.7 mg/kg intraperitoneal treatment. In the light-dark model [Crawley J.N., Pharmacol. Biochem. and Behaviour, 15, 695-699 (1981)], deramciclane showed anxiolytic activity following 3 mg/kg single subcutaneous administration. In the marble burying test considered to be experimental model of obsessive-compulsive disorder [Broekkamp C.L. et al, Eur. J. Pharmacol. 126, 223-229, (1986)], deramciclane displayed significant activity following per os administration of 10 and 30 mg/kg doses.

In the elevated plus-maze, deramciclane alone was completely inactive, however, antagonized CCK agonist induced anxiety [Gacsályi et al, Drug Dev. Res. 40, 333-348 (1997)].

The anxiolytic effects summarized above are accompanied by antidepressant effects as well. In the learned helplessness considered to be the experimental model of depression [Grial et al, Biol. Psychiatry, 23, 237-242 (1988)] deramciclane was found to be effective after intraperitoneal administration of 1 and 10 mg/kg doses.

As for receptor profile, deramciclane basically binds to central 5-HT_{2c} and 5-HT₂ₐ receptors. This unique receptor profile might explain the above summarized anxiolytic and antidepressant effects as well [Gacsályi et al, Drug Dev. Res. 40 333-348 1997)].

US-A-4 342 762 is concerned with basic ether-type compounds, namely bicyclo[2.2.1]heptane derivatives with a formula comprising also (±)-2-(2'-dimethylaminoethoxy)-2--phenyl-1,7,7-trimethyl-bicyclo(2,2,1)heptane and (±)-2-(2'--methylaminoethoxy)-2-phenyl-1,7,7-trimethyl--bicyclo(2,2,1)heptane, having tranquilizing, anti--Parkinsonian, antiepileptic, anticonvulsive, motility inhibiting, hexobarbital narcosis potentiating, analgetic effects and exhibiting antiserotonine, gastro-intestinal tract inhibiting and anti-inflammatory activities. The document is silent about the use of the compounds for the treatment of the decline/damage of the cognitive function, leave alone for the treatment or prophylaxis of Alzheimer's disease, Korsakoff syndrome, Huntington's disease or dementia.

US-A-6 093 747 is related to basic ether-type bicyclo[2.2.1]heptane derivatives with a formula comprising also (±)-2-(2'-dimethylaminoethoxy)-2-phenyl-1,7,7-trimethyl--bicyclo(2,2,1)heptane and (±)-2-(2'-methylaminoethoxy)-2--phenyl-1,7,7-trimethyl-bicyclo(2,2,1)heptane, as well, having anxiolytic effect. The document is silent about the use of the compounds for the treatment of the decline/damage of the cognitive function, leave alone for the treatment or prophylaxis of Alzheimer's disease, Korsakoff syndrome, Huntington's disease or dementia.

H. Kanerva, O. Kilkku, A. Helminen., J. Rouru and co-workers: Pharmacokinetics and safety of deramciclane during multiple oral dosing (Int. J. Clin. Pharm. And Ther. 37 (12) pages 589 to 597 [1999]) discloses the results of pharmacokinetics and safety studies carried out with deramciclane after multiple oral dosing. It refers to deramciclane as an anxiolytic pharmaceutical active ingredient. The document is silent about the use of the compounds for the treatment of the decline/damage of the cognitive function, leave alone for the treatment or prophylaxis of Alzheimer's disease, Korsakoff syndrome, Huntington's disease or dementia.

H. Kanerva, O. Kilkku, E. Heinonen, A. Helminen and co-workers: The single dose pharmacokinetics and safety of deramciclane in healthy male volunteers. (Biopharm. Drug Dispos. 20, pages 327 to 334 [1999]) either discloses the results of pharmacokinetics and safety studies carried out with deramciclane, which is recognized as an anxiolytic compound. The document is silent about the use of the compounds for the treatment of the decline/damage of the cognitive function, leave alone for the treatment or prophylaxis of Alzheimer's disease, Korsakoff syndrome, Huntington's disease or dementia.

The present invention is directed to the use of a bicycloheptane derivative for the preparation of medicaments for the treatment or prophylaxis of Alzheimer's disease, Korsakoff syndrome, Huntington's disease or dementia as well as of mental disability consequent on them.

Basically, two types of memory functions are known to exist according to the literature. In the first type, known as short term memory, the learned information is stored for a period between some minutes and a few hours. In the second type, known as long term memory, the information may be kept from hours to years [Baddley and Warington, J. Verb. Learn. Verb. Behav. 9, 176-179 (1970)]; Wright et al, Science 229, 287-289 (1985)] .

The process of information being transferred from short term memory to long term memory is called memory consolidation.

The process of recalling or manifesting information from either short term or long term memory is called memory retention. Total amnesia is relatively rare, however, diseases accompanied by various levels of memory impairment occur in a continuously growing number. At present, approximately 18 million patients are suffering from Alzheimer's disease and this number will be doubled in the next 25 years for this disease only [Fletcher, Mol. Med. Today, 3/10 p. 429-434, (1997)].

### Essence of the invention

It is the object of the present invention to develop a new use of bicyclo[2.2.1]heptane derivatives for the preparation of medicaments for treating diseases or conditions accompanied by various degrees of memory dysfunction.

The above object is achieved by means of the present invention.

The present invention is based on the recognition that the bicycloheptane derivatives of general Formula 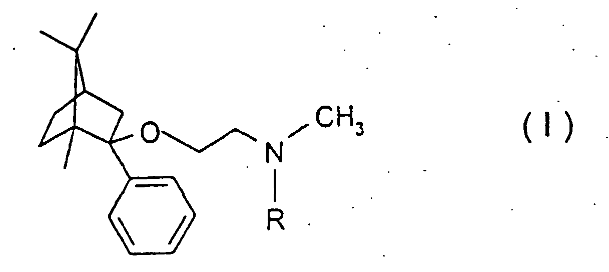 (wherein R stands for hydrogen or methyl) can be efficiently used for the preparation of medicaments for the treatment or prophylaxis of diseases characterized either by the decline and/or damage of cognitive functions.

Within the above indications, according to the invention the compounds of general Formula I are used for the preparation of medicaments for treating or preventing Alzheimer's disease, Korsakoff syndrome, Huntington's disease or dementia, in particular senile dementia of the elderly, as well as of mental disability consequent on them.

According to an embodiment of the present invention there are provided for the said medicaments in the form of pharmaceutical compositions containing the compound of Formula I or its acid addition salt in admixture with inert solid or liquid pharmaceutical carriers and/or auxiliary agents.
They can be prepared by admixing the active ingredient prepared in a known manner with pharmaceutical carriers and/or diluents and finishing in the form of pharmaceutical compositions for the treatment or prophylaxis of the said diseases.

### Details of the invention

Within the context of the present invention the general Formula I encompasses all optical isomers and mixtures thereof.

As compound of the general Formula I preferably (1R,2S,4R)--(-)-2-(2-dimethylaminoethoxy)-2-phenyl-1,7,7--trimethylbicyclo[2.2.1]heptane or pharmaceutically acceptable acid addition salts thereof can be used.

The term "pharmaceutically acceptable acid addition salt" relates to salts formed with pharmaceutically acceptable inorganic acids (e.g. hydrogen halides, such as hydrochloric acid or hydrogen bromide; sulphuric acid, nitric acid or phosphoric acid etc.) or organic acids (e.g. tartaric acid, succinic acid, malic acid, lactic acid, citric acid, maleic acid, fumaric acid, methanesulfonic acid, p-toluene-sulfonic acid etc.). According to a particularly preferred embodiment of the present invention a fumarate is used.

According to the most preferable embodiment of the present invention (1R,2S,4R)-(-)-2-(2-dimethylaminoethoxy)-2-phenyl--1,7,7-trimethyl-bicyclo[2.2.1]heptane-fumarate is used.

According to a further aspect of the present invention there is provided a pharmaceutical composition for the treatment or prophylaxis of the said diseases comprising as active ingredient a compound of the general Formula I (wherein R stands for hydrogen or methyl) or a pharmaceutically acceptable acid addition salt thereof in admixture with suitable inert solid or liquid pharmaceutical carriers and/or auxiliary agents.

The daily dosage of the compounds of the general Formula I depends on the circumstances of the given case (e.g. body weight, age and condition of the patient, the disease to be treated, the severity of the damage etc.). The daily dose of the compound of the general Formula I is generally 0.01-10 mg/kg, preferably 0.1-1.0 mg/kg.

The pharmaceutical compositions used according to the present invention can be prepared by methods of the pharmaceutical industry known per se.

For oral administration tablets, dragées, enterosolvent tablets or dragées or hard or soft gelatine capsules can be used. The active ingredient content of such compositions may be 10-100 mg per dosage unit. The above pharmaceutical compositions can contain carriers (preferably silicic acid), binders (e.g. polyvinylpyrrolidone or gelatine), sliding agents, lubricants (e.g. magnesium stearate, talc or sodium lauryl sulphate). Oral aqueous suspensions and/or elixirs can be prepared by admixing the active ingredient with taste-improving agents, dyes, emulsifiers and diluents (e.g. water, ethanol, propylenglycol, glycerol etc.).

Tablets can be prepared by dry or wet granulating methods. In course of the preparation of dragées the dragée core is coated with a suitable coating layer. Capsules are prepared by filling the mixture into hard or soft gelatine capsules.

Further details of the present invention are to be found in the following Examples.

### EXAMPLE 1

### Determination of efficacy displayed by the compounds of general Formula I for treating or preventing diseases characterized either by the decline and/or damage of cognitive functions, or mental disability accompanying other diseases.

(1 R,2S,4R)-(-)-2-(2-dimethylaminoethoxy)-2-phenyl--1,7,7-trimethyl-bicyclo[2.2.1]heptane-fumarate (deramciclane) was employed as test substance.

### Method

Male Wistar rats weighing 200-220 g were used. The animals were obtained from Charles River Co. They were kept in a room with normal 12-12 h light dark cycle (light on: 06:00) at a relative humidity of 60±10 %.

The experiment was performed in a five-channel "step through"-type passive avoidance learning apparatus. The equipment consisted of two adjacent Plexi-glass boxes of 20x20x16 cm. One of them was made of regular transparent Plexi-glass and the other one was made of black, non-transparent Plexi-glass. The boxes were connected with a 7.5x8 cm passage way, equipped with a computer-controlled guillotine-door. The passage of the rats through the door was detected by infrared photocells arranged in two parallel lines in the opening of the passage way. The door was automatically closed when the animals passed through. The dark compartment was equipped with stainless steel grid floor through which electric foot shocks could be delivered to the animals. A 10 W light bulb was installed above the passage way in the light compartment.

The experiment was performed on two consecutive days, in two sessions which were 24 h apart from each other.

On Day 1 (Acquisition) the animals acquired information about the situation (grid floor shock in the dark compartment), on Day 2 (Retention) they recalled the acquired information to avoid punishment ("if I go into the dark I will be punished, so I stay outside in the light").

### Day 1 (Acquisition)

The individually numbered animals were placed into the light compartment of the equipment. After 30 s the guillotine door was opened and the rats could freely pass to the dark (considered as safe) compartment. Step through latency was automatically determined. (Step through latency is the time period spanning from door opening to the time when the animal passed into the dark compartment.) The door was closed then, and the timer was automatically stopped. An electric foot shock of 1.2 mA lasting 2.5 s was applied to the animal through the grid floor 3 s after the door has been closed, except for rats in the absolute control group (no shock + vehicle treated). Test animals were removed from the dark compartment immediately after foot shock has been delivered. The function of the absolute control group was to shown that shocked animals will remember to the unpleasant foot shock as revealed by increased latency time when compared to absolute control. That is the essence of acquisition.

### Day 2 (Retention)

After 24 h the animals were placed again in the light compartment of the test apparatus and step-through latency was measured as described at Acquisition day, except that no foot shock was applied to the animals in any group on the second day. A maximum of 180 s time interval was available for the rats to pass into the dark compartment. The animals were removed from the light compartment if they did not pass to the dark compartment within the 180 s test period.

### Treatments

For examining the impact on learning the animals were treated *intraperitoneally* in a volume of 1 ml/kg either with (1R,2S,4R)-(-)-2-(2-dimethylaminoethoxy)-2-phenyl-1,7,7--trimethylbicyclo[2.2.1]heptane-fumarate or with vehicle (0.4 % methyl-cellulose) on Day 1, 30 min before Acquisition. When effect on retention from long term memory was investigated the animals were treated *intraperitoneally* in a volume of 1 ml/kg on Day 2, 30 min before testing Retention. Data were analyzed using two way ANOVA, followed by Duncan's post hoc test for between group differences.

### Results and Discussion

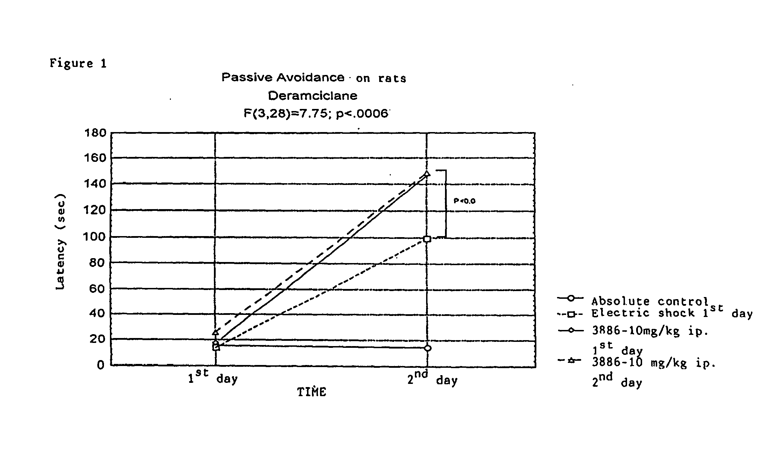

It has been surprisingly found that (1 R,2S,4R)-(-)-2-(2--dimethylaminoethoxy)-2-phenyl-1,7,7-trimethyl-bicydo[2.2.1]heptane-fumarate, known for its anxiolytic effect as major CNS effect, significantly increased step-through latency into the dark compartment of the passive avoidance apparatus both after Day 1 and/or Day 2 administration of the compound (Fig. 1).

It is shown on Fig. 1 that in absolute control group (no shock, untreated), step-through latency was approximately the same on both experimental days (this means that there was nothing to recall and avoid on the second day).

In the shocked, vehicle treated control group the unavoidable 1.2 mA foot shock resulted in a significantly increased step-through latency on Day 2 when compared to absolute control. The experimental animals recalled the annoying experience (foot shock) in the dark, therefore, they passed into the dark compartment after a significantly longer time (increased latency).

In the treated groups this augmented latency was further increased following both types of treatment. After Day 1 treatment, (probably) the acquisition of the information improved significantly, while after Day 2 treatment the retention of memory improved.

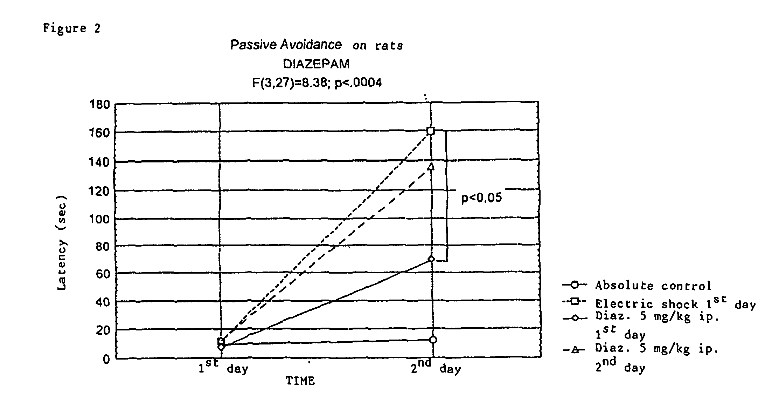

The above recognition is so much the more surprising as anxiolytic compounds either do not influence memory (i.e. buspirone) or have a deleterious effect thereon (i.e. diazepam, Fig. 2).

According to our results, 5 mg/kg ip diazepam administered on Day 1 significantly damaged retention from long term memory as shown on Fig. 2.

From the therapeutic point of view the advantageous effect of (1R,2S,4R)-(-)-2-(2-dimethylaminoethoxy)-2-phenyl--1,7,7-trimethylbicyclo[2.2.1]heptane-fumarate on learning and memory signifies that the compound could be appropriate for treating and/or preventing diseases or conditions accompanying diseases wherein learning or memory functions are suffering a loss or there is a possibility to suffering a loss. Such diseases are - as mentioned above, but not limited to - Alzheimer's disease, Korsakoff syndrome, Huntington's disease and mental decline due to aging processes or impairment of the cognitive functions due to exposure to toxic substances as well.

### EXAMPLE 2

Tablets having the following compositions are prepared by methods of pharmaceutical industry known per se.

| Component | Amount, mg/tablet |
|---|---|
| (1R,2S,4R)-(-)-2-(2-dimethylaminoethoxy)-2--phenyl-1,7,7-trimethylbicyclo[2.2.1]heptane--fumarate | 20 |
| Maize starch | 90 |
| Polyvinyl pyrrolidone | 68 |
| Magnesium stearate | 2 |
| Total weight | 180 |

### EXAMPLE 3

Gelatine capsules having the following compositions are prepared by methods of pharmaceutical industry known per se.

| Component | Amount, mg/capsule |
|---|---|
| (1R,2S,4R)-(-)-2-(2-dimethylaminoethoxy)-2--phenyl-1,7,7-trimethylbicyclo[2.2.1]heptane-fumarate | 20 |
| Maize starch | 212 |
| Aerosil^{®} | 5 |
| Magnesium stearate | 3 |
| Total weight | 240 |

## Claims

1. Use of a bicycloheptane derivative of the general Formula 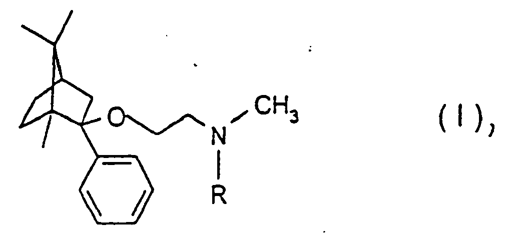 wherein R stands for hydrogen or methyl, or a pharmaceutically acceptable acid addition salt thereof as active ingredient(s) for the preparation of medicaments for the treatment or prophylaxis of Alzheimer's disease, Korsakoff syndrome, Huntington's disease or dementia as well as of mental disability consequent on them.

2. Use according to claim 1, **characterized in that** the medicaments are for the treatment or prophylaxis of Alzheimer's disease or dementia.

3. Use according to claim 1 or 2, **characterized in that** the dementia are senile dementia of the elderly.

4. Use according to claims 1 to 3, **characterized in that** the compound of Formula I is (1R,2S,4R)-(-)-2--(2-dimethylaminoethoxy)-2-phenyl-1,7,7--trimethylbicyclo[2.2.1]heptane or a pharmaceutically acceptable acid addition salt thereof.

5. Use according to claims 1 to 4, **characterized in that** the acid addition salt of the compound of Formula I is (1R,2S,4R)-(-)-2-(2-dimethylaminoethoxy)-2-phenyl-1,7,7--trimethylbicyclo[2.2.1]heptane-fumarate.

6. Use according to claims 1 to 5, **characterized in that** the medicaments are in the form of pharmaceutical compositions containing the compound of Formula I or its acid addition salt in admixture with inert solid or liquid pharmaceutical carriers and/or auxiliary agents.

## Patentansprüche

1. Verwendung eines Bicycloheptanderivats der allgemeinen Formel 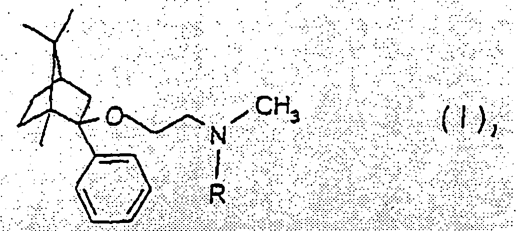 worin R für Wasserstoff oder Methyl steht, oder eines pharmazeutisch annehmbaren Säureadditionssalzes davon als Wirkstoff(e) für die Herstellung von Medikamenten zur Behandlung oder Prophylaxe von Alzheimer-Erkrankung, Korsakoff-Syndrom, Huntington-Erkrankung oder Demenz sowie von dem mentalen Abbau als Folge von diesen.

2. Verwendung gemäß Anspruch 1, **dadurch gekennzeichnet, dass** die Medikamente für die Behandlung oder Prophylaxe von Alzheimer-Erkrankung oder Demenz sind.

3. Verwendung gemäß Anspruch 1 oder 2, **dadurch gekennzeichnet, dass** die Demenz die senile Demenz von Senioren ist

4. Verwendung gemäß Anspruch 1 bis 3, **dadurch gekennzeichnet, dass** die Verbindung der Formel I (1R,2S,4R)-(-)-2-(2-Dimethylaminoethoxy)-2-phenyl-1,7,7-trimethylbicylo-[2.2.1]heptan oder ein pharmazeutisch annehmbares Säureadditionssalz davon ist.

5. Verwendung gemäß Anspruch 1 bis 4, **dadurch gekennzeichnet, dass** das Säureadditionssalz der Verbindung der Formel I (1R,2S,4R)-(-)-2-(2-Dimethylaminoethoxy)-2-phenyl-1,7,7-trimethylbicyclo[2.2.1]heptan-fumarat ist.

6. Verwendung gemäß Anspruch 1 bis 5, **dadurch gekennzeichnet, dass** die Medikamente in Form von pharmazeutischen Zusammensetzungen vorliegen, die die Verbindung der Formel I oder ihr Säureadditionssalz in Vermischung mit inerten festen oder flüssigen pharmazeutischen Trägem und/oder Hilfsstoffen enthalten.

## Revendications

1. Utilisation d'un dérivé de bicycloheptane de Formule générale 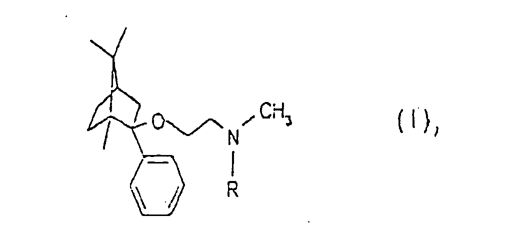 où R représente un hydrogène ou un méthyle ou un sel d'addition acide pharmaceutiquement acceptable de ceux-ci comme ingrédient(s) actif(s) pour la préparation de médicaments destinés au traitement ou à la prophylaxie de la maladie d'Alzheimer, du syndrome de Korsakoff, de la maladie de Huntington ou de la démence ainsi que des incapacités mentales qui en découlent.

2. Utilisation selon la revendication 1, **caractérisée par le fait que** les médicaments sont destinés au traitement ou à la prophylaxie de la maladie d'Alzheimer ou de la démence.

3. Utilisation selon la revendication 1 ou 2, **caractérisé par le fait que** la démence concerne la démence sénile des personnes âgées.

4. Utilisation selon les revendications 1 à 3, **caractérisée par le fait que** le composé de Formule I est (1R, 2S, 4R)-(-)-2-(2-diméthylamino-éthoxy)-2-phényl-1,7,7--triméthylbicyclo[2.2.1]heptane ou un sel d'addition acide pharmaceutiquement acceptable de ceux-ci.

5. Utilisation selon les revendications 1 à 4, **caractérisée par le fait que** le sel d'addition acide du composé de Formule I est (1R,2S,4R)-(-)-2-(2-diméthylamino-éthoxy)-2-phényl -1,7,7-triméthylbicyclo[2.2.1]heptane-fumarate.

6. Utilisation selon les revendications 1 à 5, **caractérisée par le fait que** les médicaments sont sous forme de compositions pharmaceutiques contenant le composé de Formule I ou son sel d'addition acide en mélange avec des véhicules pharmaceutiques solides ou liquides et/ou des agents auxiliaires.
